# EUROPEAN PATENT SPECIFICATION

(11) **EP 4 115 800 B1**
(45) Date of publication and mention of the grant of the patent: **30.04.2025**
(21) Application number: 22179029.8
(22) Date of filing: 14.06.2022
(51) Int. Cl.: A61B 5/0215

(54) **VENOUS PRESSURE MEASUREMENT DEVICE AND VENOUS PRESSURE MEASUREMENT METHOD**
VENENDRUCKMESSVORRICHTUNG UND VENENDRUCKMESSVERFAHREN
DISPOSITIF DE MESURE DE LA PRESSION VEINEUSE ET PROCÉDÉ DE MESURE DE LA PRESSION VEINEUSE

(30) Priority: 23.06.2021 JP 2021103861
(43) Date of publication of application: 11.01.2023
(73) Proprietor: Nihon Kohden Corporation, Shinjuku-ku Tokyo (JP)
(72) Inventor: Morimoto, Haruka, Tokorozawa-shi, Saitama (JP); Kobayashi, Naoki, Tokorozawa-shi, Saitama (JP); Motogi, Jun, Tokorozawa-shi, Saitama (JP)
(74) Representative: Grünecker Patent- und Rechtsanwälte PartG mbB

(56) References cited:
- JP-A- 2002 191 569
- JP-A- 2015 047 400
- JP-A- 2019 017 553
- JP-A- H05 207 979
- US-A1- 2013 006 123
- US-A1- 2018 028 161

## Description

### TECHNICAL FIELD

The presently disclosed subject matter relates to a venous pressure measurement device and a venous pressure measurement method.

### BACKGROUND ART

In recent years, the number of patients involved in cardiac diseases including heart failure has continued to increase, and the cardiac diseases become a major cause of death.

As the number of elderly people increases, it is expected that the number of elderly patients with heart failure will significantly increase (so-called heart failure pandemic), and it is expected that the number of patients with heart failure will reach 1.3 million in 2030.

In addition to such an increase in the number of patients, a high rehospitalization rate for heart failure is a social problem, which leads to increase in medical expenses. Such a problem occurs not only in Japan but also in various foreign countries such as the United States.

One cause of rehospitalization of patients with heart failure is exacerbation of congestion. Since venous pressure can be an indicator of body congestion, apparatuses and methods for measuring venous pressure have been developed (for example, JP-A-2012-205822).

In such venous pressure measurement, even in a case where a value of venous pressure cannot be uniquely determined, if information on a range of values that can be estimated as the venous pressure, that is, a value range of the venous pressure, can be obtained, the information can be effective for determining a state of body congestion or the like.

Therefore, an object of the presently disclosed subject matter is to provide a venous pressure measurement device and a venous pressure measurement method capable of calculating a value range of venous pressure.
JP H05 207979 A, JP 2015 047400 A, and JP 2019 017553 A disclose blood pressure measuring devices. US 2018/0028161 A1 publishes diagnostic guidance systems and methods. JP 2002 191569 A discloses a pulse rate monitor and a method for measuring pulse rate. US 2013/0006123 A1 discloses a biological information processing device.

### SUMMARY

The above problems of the presently disclosed subject matter are solved by the following configurations.

A first aspect of a venous pressure measurement device according to the presently disclosed subject matter includes: an obtaining unit configured to obtain pulse wave information on a pulse wave of a subject measured by a sensor; and a processing unit configured to calculate a value range of venous pressure of the subject based on the pulse wave information obtained by the obtaining unit when the venous pressure of the subject is not estimatable from the pulse wave information obtained by the obtaining unit.

A second aspect of a venous pressure measurement method according to the presently disclosed subject matter includes: obtaining pulse wave information on a pulse wave of a subject measured by a sensor; and calculating a value range of venous pressure of the subject based on the obtained pulse wave information when the venous pressure of the subject is not estimatable from the obtained pulse wave information. The invention is defined in the independent claims.

### BRIEF DESCRIPTION OF DRAWINGS

Exemplary embodiments of the present invention will be described in detail based on the following figures, wherein:
FIG. 1 illustrates an example of an overall configuration of a venous pressure measurement system according to an embodiment of the presently disclosed subject matter;
FIG. 2 is a block diagram illustrating an example of a configuration of a venous pressure measurement device illustrated in FIG. 1;
FIG. 3 is a block diagram illustrating an example of functions of a CPU illustrated in FIG. 2;
FIG. 4 illustrates an example of pressure information obtained by a venous pressure measurement device 160 illustrated in FIG. 1 and an example of pulse wave information;
FIG. 5 illustrates another example of the pressure information obtained by the venous pressure measurement device 160 illustrated in FIG. 1 and another example of the pulse wave information;
FIG. 6 illustrates another example of the pressure information obtained by the venous pressure measurement device 160 illustrated in FIG. 1 and another example of the pulse wave information;
FIG. 7 illustrates another example of the pressure information obtained by the venous pressure measurement device 160 illustrated in FIG. 1 and another example of the pulse wave information;
FIG. 8 illustrates another example of the pressure information obtained by the venous pressure measurement device 160 illustrated in FIG. 1 and another example of the pulse wave information;
FIG. 9 illustrates another example of the pressure information obtained by the venous pressure measurement device 160 illustrated in FIG. 1 and another example of the pulse wave information;
FIG. 10 illustrates another example of the pressure information obtained by the venous pressure measurement device 160 illustrated in FIG. 1 and another example of the pulse wave information;
FIG. 11 is a flowchart illustrating an example of processing of the venous pressure measurement device illustrated in FIG. 1; and
FIG. 12 is a flowchart illustrating another example of the processing of the venous pressure measurement device illustrated in FIG. 1.

### DESCRIPTION OF EMBODIMENTS

Hereinafter, a venous pressure measurement device according to an embodiment of the presently disclosed subject matter will be described in detail with reference to the drawings.

In the drawings, the same members are denoted by the same reference numerals. In addition, dimensional ratios in the drawings are exaggerated for convenience of description, and may be different from actual ratios.

### <Embodiment>

### (Configuration of Venous Pressure Measurement System)

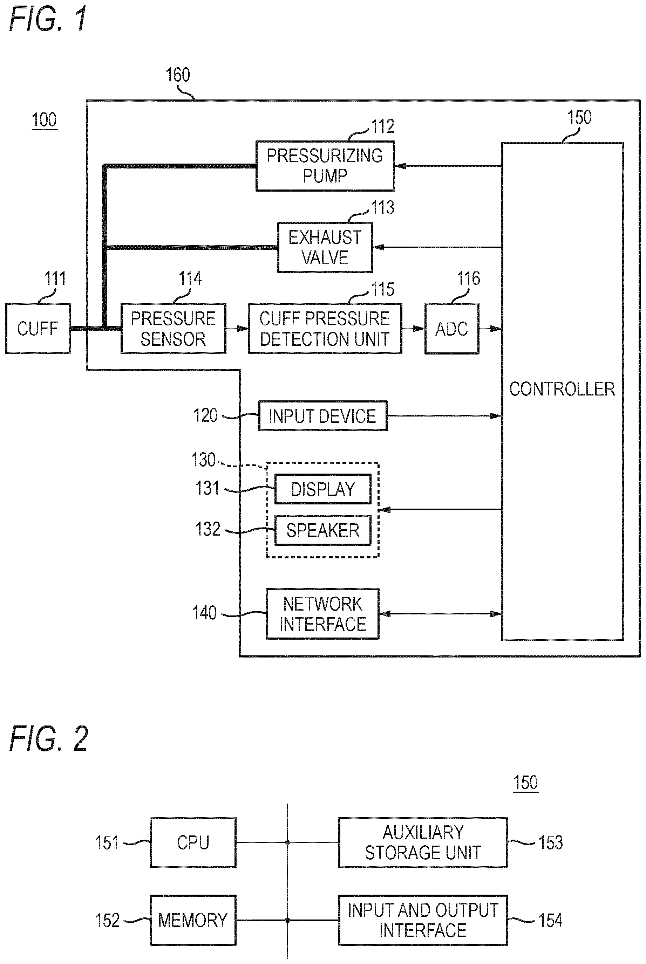

FIG. 1 is a block diagram illustrating a schematic hardware configuration of a venous pressure examination system 100 according to the present embodiment.

FIG. 2 is a block diagram illustrating a schematic hardware configuration of a controller 170.

The venous pressure examination system 100 is a system that uniquely estimates venous pressure of a subject or calculates a value range of the venous pressure. The venous pressure is an index reflecting pressure measured in a right atrium (right atrial pressure), and reflects an amount of blood returning to a heart and a preload of the right atrium.

As illustrated in FIG. 1, the venous pressure examination system 100 can include a cuff 111 and a venous pressure measurement device 160. The cuff 111 is configured to be connectable to the venous pressure measurement device 160. The venous pressure measurement device 160 can include a pressurizing pump 112, an exhaust valve 113, a pressure sensor 114, a cuff pressure detection unit 115, an AD converter (ADC) 116, an input device 120, an output device 130, a network interface 140, and a controller 150. In the venous pressure examination system 100, a principle of an oscillometric method is used. Specifically, when pressure applied to an upper arm or the like of the subject by the cuff 111 is the same as venous pressure of the subject, a maximum amplitude of a waveform of a pulse wave detected by the pressure sensor 114 is used.

The cuff 111, the pressurization pump 112, the exhaust valve 113, the pressure sensor 114, the cuff pressure detection unit 115, and the ADC 116 are an example of a configuration that performs a sensing process of measuring a venous wave of the subject, which constitutes a sensor.

The cuff 111 is an air bladder wound around the upper arm of the subject. The pressurizing pump 112 sends air to the cuff 111 and increases pressure in the air bladder (hereinafter referred to as the "cuff internal pressure") in accordance with an instruction from the controller 150. As a result, pressure applied to the upper arm of the subject by the cuff 111 (hereinafter referred to as the "cuff pressure") can be increased. The exhaust valve 113 opens the air in the air bladder to the atmosphere so as to gradually exhaust the air from the cuff 111, and thus decreases the cuff internal pressure. As a result, the cuff pressure can be decreased.

The pressure sensor 114 detects the cuff internal pressure. A pulse wave (hereinafter referred to as the "cuff pulse wave") of the subject in the process of increasing and decreasing the cuff pressure is superimposed on the cuff internal pressure. The cuff pulse wave may be a waveform in which the venous wave and an arterial wave of the subject are superimposed. The cuff pressure detection unit 115 extracts the cuff pulse wave superimposed on the cuff internal pressure from the detected cuff internal pressure, and outputs the cuff internal pressure and the extracted cuff pulse wave as analog signals to the ADC 116. The ADC 116 converts the analog signals of the cuff internal pressure and the cuff pulse wave into digital signals and transmits the digital signals to the controller 150. It should be noted that the pressure sensor 114, the cuff pressure detection unit 115, and the ADC 116 may also be integrated with the cuff 111. Alternatively, the pressure sensor 114 and the cuff pressure detection unit 115 may be integrated with the cuff 111 while the ADC 116 may be provided in the venous pressure measurement device 160.

The input device 120 receives an input operation performed by a user who operates the venous pressure measurement device 160, and generates an input signal corresponding to the input operation. The input device 120 can include, for example, a touch panel overlaid on a display 131 of the output device 130, an operation button, a mouse, a keyboard, or the like attached to a housing of the venous pressure measurement device 160. The input signal generated by the input device 120 is transmitted to the controller 150. The controller 150 executes predetermined processing corresponding to the input signal.

The output device 130 outputs an estimation result of the venous pressure of the subject or a calculation result of the value range of the venous pressure. The output device 130 can include the display 131 and a speaker 132. The display 131 may be a liquid crystal display, an organic EL display, or the like attached to the housing of the venous pressure measurement device 160. The display 131 may be a display device such as a transmissive or non-transmissive head-mounted display mounted on a head of the user.

The speaker 132 is attached to the housing of the venous pressure measurement device 160, and can output the estimation result of the venous pressure or the calculation result of the value range of the venous pressure by voice.

The output device 130 is not limited to the display 131 and the speaker 132, and may be, for example, a printer configured to print and output the estimation result of the venous pressure or the calculation result of the value range of the venous pressure.

The network interface 140 is configured to connect the controller 150 to a communication network. Specifically, the network interface 140 can include various interface processing circuits configured to communicate with an external device via the communication network, and is configured to conform to a communication standard for communicating via the communication network. The communication network is a local area network (LAN), a wide area network (WAN), the Internet, or the like. The network interface 140 may also be a wireless communication interface such as Bluetooth (registered trademark) or near field communication (NFC).

The controller 150 estimates the venous pressure of the subject or calculates the value range of the venous pressure of the subject. The controller 150 may be software and hardware that mainly control the venous pressure measurement device 160, or the controller 150 may be an independent device. For example, the controller 150 may be a dedicated medical device that examines venous pressure, or may be a personal computer, a smartphone, a tablet terminal, or the like in which a program for examining venous pressure is installed. Further, the controller 150 may also be a wearable device or the like attached to a body (for example, the upper arm or the like) of the subject.

Details of functions of the controller 150 will be described later.

As illustrated in FIG. 2, the controller 150 can include a central processing unit (CPU) 151, a memory 152, an auxiliary storage unit 153, and an input and output interface 154.

The memory 152 may include a read only memory (ROM) and a random access memory (RAM). Various programs, various parameters, and the like are stored in the ROM. In addition, the RAM can include a work area in which various programs to be executed by the CPU 151 are stored. The CPU 151 is configured to load a program specified from various programs stored in the ROM or the auxiliary storage unit 153 onto the RAM and execute various processes in cooperation with the RAM.

The auxiliary storage unit 153 may include, for example, a storage device (storage) such as a hard disk drive (HDD), a solid state drive (SSD), or a USB flash memory. The auxiliary storage unit 153 stores various programs and various types of data. In addition, the auxiliary storage unit 153 stores the estimation result of the venous pressure or the calculation result of the value range of the venous pressure.

The input and output interface 154 functions as an interface between the CPU 151 and the input device 120 and the output device 130. The input and output interface 154 can include various communication modules that communicate with input devices such as a mouse and a keyboard, a drive module that drives the display 131 and the speaker 132, and the like.

When the CPU 151 executes a program, the controller 150 controls each unit of the venous pressure examination system 100 to implement various functions.

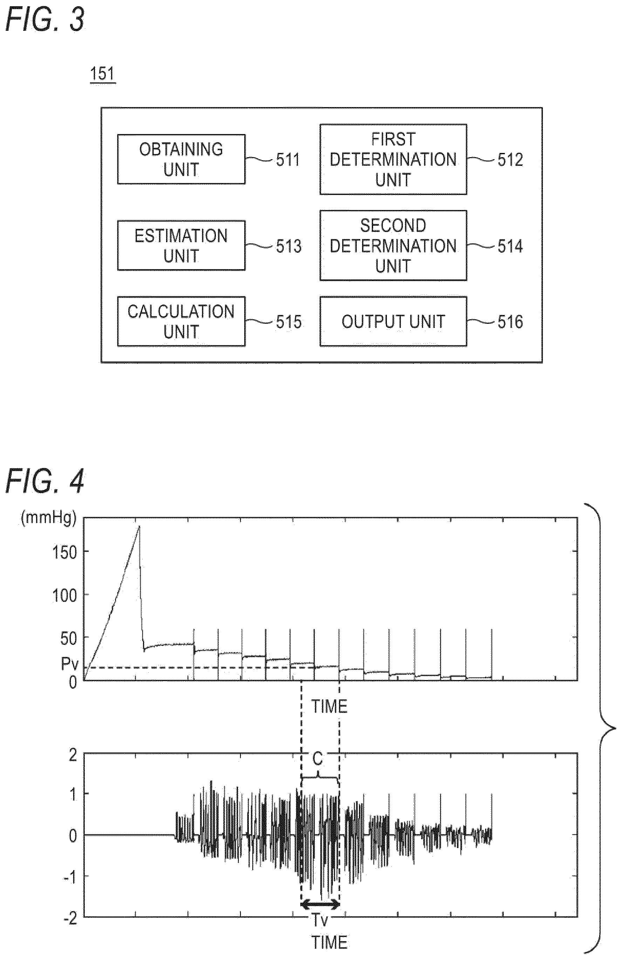

FIG. 3 is a block diagram illustrating functions of the controller 150. The controller 150 functions as, for example, an obtaining unit 511, a first determination unit 512, an estimation unit 513, a second determination unit 514, a calculation unit 515, and an output unit 516. Here, the first determination unit 512, the estimation unit 513, the second determination unit 514, and the calculation unit 515 correspond to a specific example of a "processing unit" of the presently disclosed subject matter.

The obtaining unit 511 obtains pulse wave information on the cuff pulse wave of the subject. The pulse wave information is, for example, a waveform of the cuff pulse wave obtained from the cuff pressure detection unit 115 via the ADC 116. The obtaining unit 511 further obtains pressure information on the cuff pressure. The pressure information is, for example, a value of the cuff pressure at each time.

The first determination unit 512 determines whether the venous pressure of the subject can be estimated from the pulse wave information obtained by the obtaining unit 511. For example, the first determination unit 512 determines that the venous pressure of the subject cannot be estimated when it is determined that the pulse wave information does not include a candidate component that can be estimated as the venous wave of the subject (for example, a candidate component C in a lower graph of FIG. 4 to be described later). Whether the pulse wave information includes the candidate component is determined based on, for example, a change in an amplitude of the waveform of the cuff pulse wave.

An upper graph of FIG. 4 illustrates an example of the pressure information obtained by the obtaining unit 511, and the lower graph of FIG. 4 illustrates the pulse wave information corresponding to the pressure information illustrated in the upper graph of FIG. 4. Specifically, the upper graph of FIG. 4 illustrates a change over time in the value of the cuff pressure, and the lower graph of FIG. 4 illustrates the waveform of the cuff pulse wave corresponding to the cuff pressure of the upper graph of FIG. 4.

Here, a method in which the venous pressure examination system 100 uniquely estimates the venous pressure of the subject will be described.

As the cuff pressure decreases in a stepwise manner (stepwise), an arterial pulse wave component superimposed on the cuff pressure decreases while a venous pulse wave component gradually increases accordingly. When the cuff pressure further decreases, the venous pulse wave component further increases. When the cuff pressure becomes equal to blood pressure applied to a blood vessel wall of a vein of an upper arm of a patient, the amplitude of the waveform of the cuff pulse wave becomes maximum. Therefore, the venous pressure examination system 100 estimates the cuff pressure (for example, Pv of the upper graph of FIG. 4) at the time when the amplitude of the waveform of the cuff pulse wave changes in a predetermined manner, for example, at the time when the amplitude of the waveform of the cuff pulse wave becomes maximum, as the venous pressure of the subject.

In the example illustrated in the upper and lower graphs of FIG. 4, as the cuff pressure gradually decreases from diastolic pressure of arterial blood pressure of the subject (the upper graph of FIG. 4), the amplitude of the waveform of the cuff pulse wave gradually decreases and then gradually increases again, and becomes maximum in a period Tv and then gradually decreases (the lower graph of FIG. 4). That is, a tendency of the amplitude change is switched before and after the period Tv. At this time, for example, the first determination unit 512 determines that the venous pressure of the subject can be uniquely estimated since the pulse wave information includes the candidate component C of the period Tv that can be estimated as the venous wave of the subject and does not include any noise component affected by disturbance.

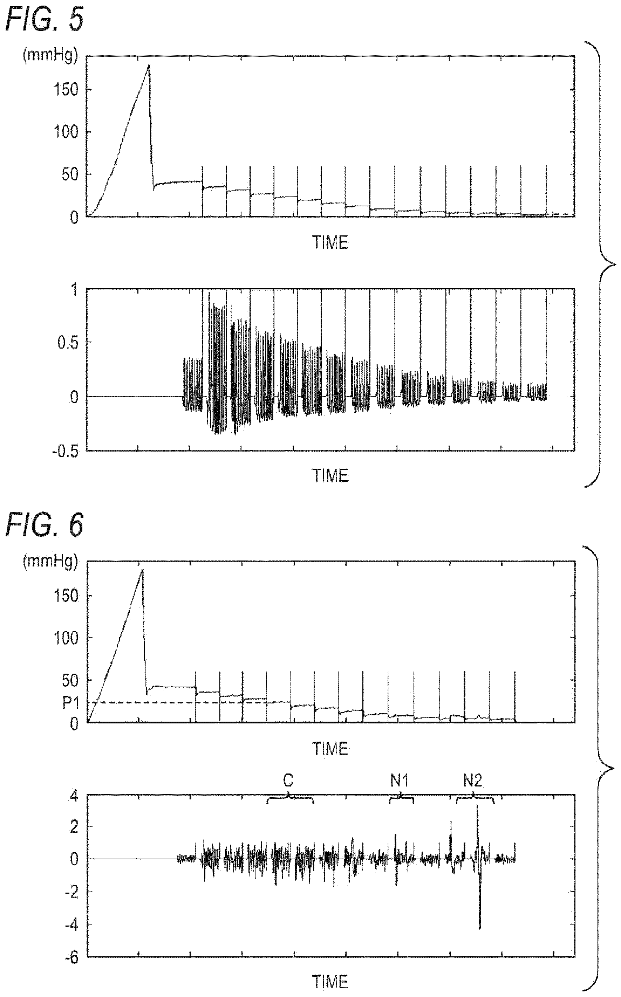

An upper graph of FIG. 5 illustrates another example of the pressure information obtained by the obtaining unit 511, and a lower graph of FIG. 5 illustrates the pulse wave information corresponding to the pressure information illustrated in the upper graph of FIG. 5. At this time, as the cuff pressure gradually decreases from the diastolic pressure of the arterial blood pressure of the subject (the upper graph of FIG. 5), the amplitude of the waveform of the cuff pulse wave continues to decrease. That is, in the waveform of the cuff pulse wave, the tendency of the amplitude change is not switched. At this time, for example, the first determination unit 512 determines that the venous pressure of the subject cannot be estimated since the pulse wave information does not include the candidate component that can be estimated as the venous wave of the subject.

When the pulse wave information includes the noise component that is affected by disturbance and satisfies a predetermined condition, the first determination unit 512 determines that the venous pressure of the subject cannot be estimated. This is because, if the noise component is included, the noise component and a component derived from the pulse wave of the subject overlap with each other, and thus accurate information on the pulse wave of the subject cannot be obtained. Even if the pulse wave information includes the noise component, the first determination unit 512 may determine that the venous pressure of the subject can be estimated when an influence on venous pressure measurement is small. For example, when the pulse wave information includes a noise component having a predetermined or greater magnitude, a predetermined number or more noise components, or a noise component lasting for a predetermined or longer period, the first determination unit 512 determines that the venous pressure of the subject cannot be estimated. The first determination unit 512 may determine that the venous pressure of the subject can be estimated according to a position (for example, cuff pressure at which the noise component appears), a period length, a number, a magnitude, and the like of the noise component included in the pulse wave information.

The first determination unit 512 determines whether the pulse wave information includes the noise component satisfying the predetermined condition, for example, by performing frequency analysis. Alternatively, the first determination unit 512 may determine whether the pulse wave information includes the noise component satisfying the predetermined condition based on a change rate, a magnitude, or the like of the amplitude of the waveform of the cuff pulse wave. For example, the first determination unit 512 may determine that the pulse wave information includes the noise component satisfying the predetermined condition when there is a rapid amplitude change as compared with a waveform of an immediately preceding beat, or may determine that the pulse wave information includes the noise component satisfying the predetermined condition when there is an amplitude greater than a predetermined amplitude. The first determination unit 512 may determine whether the pulse wave information includes the noise component satisfying the predetermined condition based on information obtained from an external sensor. For example, an acceleration sensor or the like can be used as the external sensor. The first determination unit 512 may determine whether the pulse wave information includes the noise component satisfying the predetermined condition through using a machine learning model.

Upper graphs of Figs. 6-10 illustrate other examples of the pressure information obtained by the obtaining unit 511, and lower graphs of Figs. 6-10 illustrate the pulse wave information corresponding to the pressure information illustrated in the upper graphs of Figs. 6-10, respectively.

When the obtaining unit 511 obtains the pressure information and the pulse wave information illustrated in the upper and lower graphs of Fig. 6, the first determination unit 512, for example, determines that the venous pressure of the subject cannot be estimated since the pulse wave information includes the candidate component C and noise components N1 and N2.

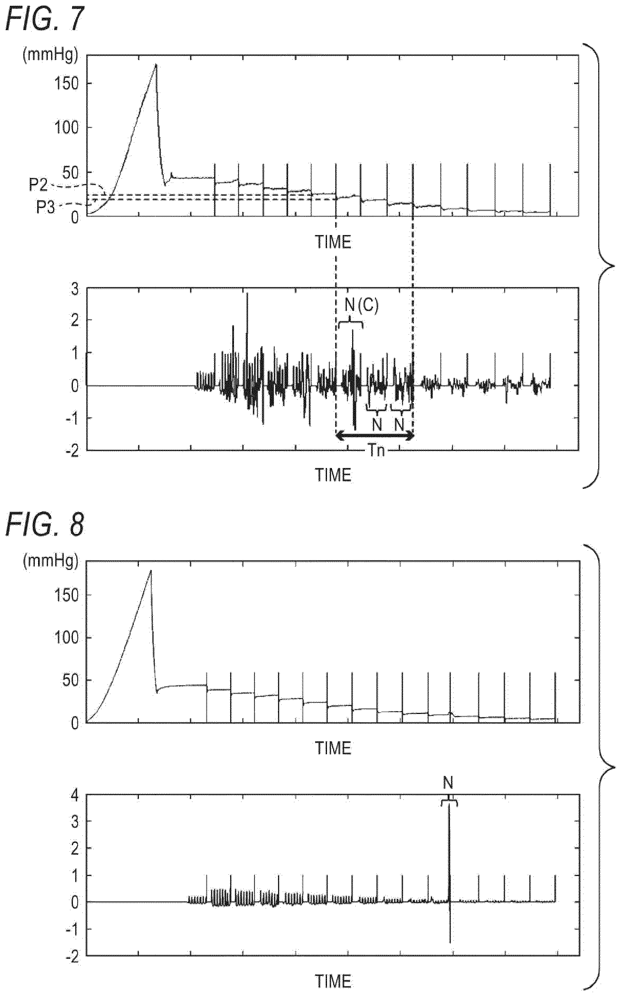

When the obtaining unit 511 obtains the pressure information and the pulse wave information illustrated in the upper and lower graphs of Fig. 7, the first determination unit 512 determines that the venous pressure of the subject cannot be estimated since the pulse wave information includes a noise component N of a period Tn.

When the obtaining unit 511 obtains the pressure information and the pulse wave information illustrated in the upper and lower graphs of Fig. 8, the first determination unit 512, for example, determines that the venous pressure of the subject cannot be estimated since the pulse wave information includes the noise component N.

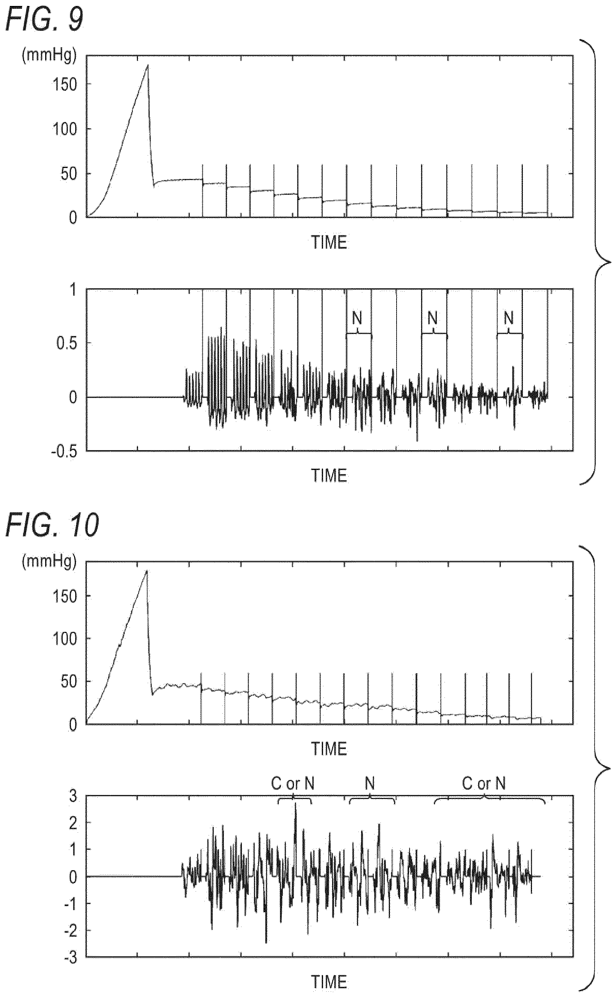

When the obtaining unit 511 obtains the pressure information and the pulse wave information illustrated in the upper and lower graphs of Fig. 9, the first determination unit 512, for example, determines that the venous pressure of the subject cannot be estimated since the pulse wave information includes the noise component N.

When the obtaining unit 511 obtains the pressure information and the pulse wave information illustrated in the upper and lower graphs of Fig. 10, the first determination unit 512, for example, determines that the venous pressure of the subject cannot be estimated since the pulse wave information includes the candidate component C and the noise component N.

When the first determination unit 512 determines that the venous pressure of the subject can be estimated, the estimation unit 513 estimates the venous pressure of the subject based on the pulse wave information obtained by the obtaining unit 511. Specifically, cuff pressure at which the amplitude of the waveform of the cuff pulse wave becomes maximum is estimated as the venous pressure of the subject.

For example, when the pressure information and the pulse wave information illustrated in the upper and lower graphs of FIG. 4 are obtained by the obtaining unit 511, the estimation unit 513 estimates the cuff pressure Pv in the period Tv as the venous pressure of the subject.

When the first determination unit 512 determines that the venous pressure of the subject cannot be estimated, the second determination unit 514 determines whether the value range of the venous pressure of the subject can be calculated based on the pulse wave information obtained by the obtaining unit 511. The second determination unit 514 determines whether the value range of the venous pressure of the subject can be calculated, for example, according to at least one of the position (for example, the cuff pressure at which the noise component appears), the period length, the number, and the magnitude of the noise component included in the pulse wave information. For example, the second determination unit 514 determines that the value range of the venous pressure cannot be calculated when the number, the magnitude, or the like of the noise component exceeds a predetermined value, and determines that the value range of the venous pressure can be calculated when the number, the magnitude, or the like of the noise component is equal to or smaller than the predetermined value. The second determination unit 514 may perform the determination based on a requirement other than the position, the period length, the number, or the magnitude of the noise component, or may perform the determination based on a component other than the noise component.

When the first determination unit 512 determines that the venous pressure of the subject cannot be estimated and the second determination unit 514 determines that the value range of the venous pressure of the subject can be calculated, the calculation unit 515 calculates the value range of the venous pressure of the subject based on the pulse wave information obtained by the obtaining unit 511. When the first determination unit 512 determines that the venous pressure of the subject cannot be estimated and the second determination unit 514 determines that the value range of the venous pressure of the subject cannot be calculated, the calculation unit 515 does not calculate the value range of the venous pressure of the subject.

For example, when the obtaining unit 511 obtains the pressure information and the pulse wave information illustrated in the upper and lower graphs of FIG. 5, as described above, the first determination unit 512 determines that the pulse wave information does not include the candidate component and thus the venous pressure of the subject cannot be estimated. Meanwhile, the second determination unit 514 determines that the value range of the venous pressure of the subject can be calculated since the pulse wave information does not include any noise component satisfying the predetermined condition. At this time, the calculation unit 515 calculates, for example, a range outside a range of the measured cuff pressure as the value range of the venous pressure of the subject. Specifically, in the upper and lower graphs of FIG. 5, the cuff pressure decreases from the diastolic pressure of the arterial blood pressure to 3 mmHg. A dashed line in a right end of the upper graph represents 3mmHg. The calculation unit 515 determines the value range of the venous pressure of the subject is lower than 3 mmHg.

For example, when the obtaining unit 511 obtains the pressure information and the pulse wave information illustrated in the upper and lower graphs of FIG. 6, as described above, the first determination unit 512 determines that the venous pressure of the subject cannot be estimated since the pulse wave information includes the candidate component C and the noise components N1 and N2. The candidate component C is detected when the cuff pressure is P1, and the noise components N1 and N2 are detected when the cuff pressure is lower than P1. Meanwhile, the second determination unit 514 determines that the value range of the venous pressure of the subject can be calculated based on positions, period lengths, numbers, magnitudes, and the like of the noise components N1 and N2. At this time, the calculation unit 515 calculates the value range of the venous pressure of the subject by using, for example, the pulse wave information and the pressure information in a range including the candidate component C and the noise components N1 and N2. Specifically, the calculation unit 515 determines that the value range of the venous pressure of the subject is equal to or lower than P1.

When the obtaining unit 511 obtains the pressure information and the pulse wave information illustrated in the upper and lower graphs of FIG. 7, as described above, the first determination unit 512 determines that the venous pressure of the subject cannot be estimated since the pulse wave information includes the noise component N of the period Tn. The noise component N is detected when the cuff pressure is P3. Meanwhile, the second determination unit 514 determines that the value range of the venous pressure of the subject can be calculated based on a position, a period length, a number, a magnitude, and the like of the noise component N. In the lower graph of FIG. 7, an amplitude of a waveform immediately after the period Tn is greater than an amplitude of a waveform immediately before the period Tn. At this time, the calculation unit 515, for example, estimates that the waveform of the cuff pulse wave in the period Tn includes the candidate component C together with the noise component N based on the amplitude change before and after the period Tn, and calculates the value range of the venous pressure of the subject. Specifically, the calculation unit 515 determines that the value range of the venous pressure of the subject is equal to or higher than P3. Alternatively, the calculation unit 515 may determine that the value range of the venous pressure of the subject is equal to or higher than P3 and equal to or lower than P2 through using a cuff pressure P2 immediately before the period Tn.

When the obtaining unit 511 obtains the pressure information and the pulse wave information illustrated in the upper and lower graphs of FIG. 8, as described above, the first determination unit 512 determines that the venous pressure of the subject cannot be estimated since the pulse wave information includes the noise component N. Meanwhile, the second determination unit 514 determines that the value range of the venous pressure of the subject can be calculated based on the position, the period length, the number, the magnitude, and the like of the noise component N. At this time, the calculation unit 515 calculates, for example, a range outside the range of the measured cuff pressure as the value range of the venous pressure of the subject.

When the obtaining unit 511 obtains the pressure information and the pulse wave information illustrated in the upper and lower graphs of FIG. 9 and FIG. 10, as described above, the first determination unit 512 determines that the venous pressure of the subject cannot be estimated since the pulse wave information includes the noise component N. In addition, the second determination unit 514 determines that the value range of the venous pressure of the subject cannot be calculated based on the position, the period length, the number, the magnitude, and the like of the noise component N (the upper and lower graphs of FIG. 9). In the upper and lower graphs of FIG. 10, although the candidate component C is included in the pulse wave information together with the noise component N, since the waveform is not stable, the second determination unit 514 determines that the value range of the venous pressure of the subject cannot be calculated.

For example, the output unit 516 outputs, to an output device 60, any one of the following: information on the estimation result estimated by the estimation unit 513; information on the calculation result calculated by the calculation unit 515; and information on an error result indicating that the estimation of the venous pressure of the subject and the calculation of the value range thereof cannot be performed. The output device 60 displays, for example, the venous pressure of the subject estimated by the estimation unit 513 or the value range of the venous pressure of the subject calculated by the calculation unit 515. The output device 60 may display, for example, a level of the venous pressure of the subject relative to a predetermined value. When the estimated venous pressure of the subject is higher or lower than the predetermined value, an alarm display or an alarm sound may be output. The output device 60 may display the error result indicating that the venous pressure of the subject cannot be estimated and the value range thereof cannot be calculated, and for example, an error display or an error sound may be output.

### (Processing (Measurement) Method of Venous Pressure Measurement Device)

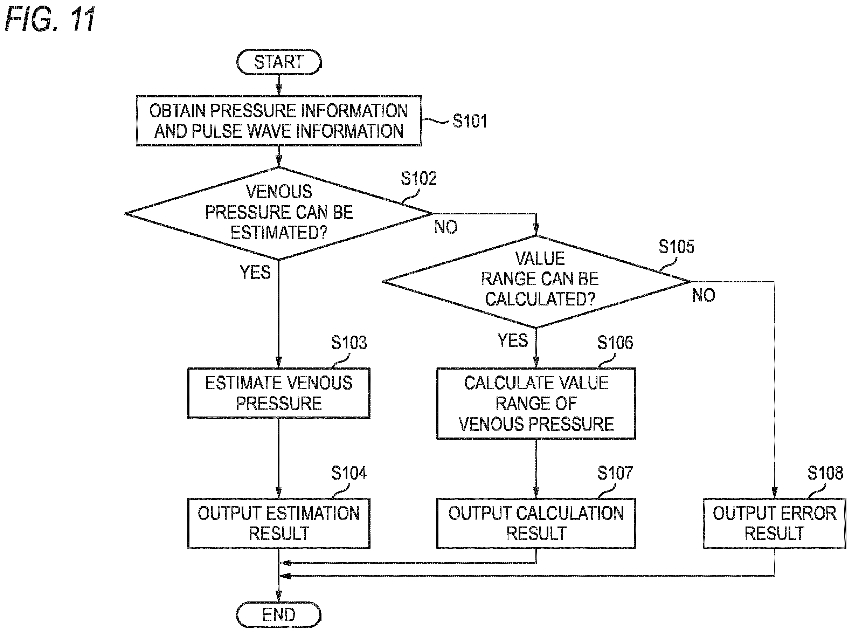

FIG. 11 is a flowchart illustrating an example of processes performed by the venous pressure measurement device 160, in other words, a venous pressure measurement method performed by the venous pressure measurement device 160.

First, the venous pressure measurement device 160 obtains the pulse wave information and the pressure information (step S101). The pulse wave information is obtained, for example, as follows.

First, the cuff 111 is attached to the upper arm of the subject, and the cuff pressure is increased by the pressurizing pump 112. Next, the cuff pressure is decreased to pressure equal to or lower than diastolic blood pressure of the subject (for example, about 40 mmHg to 50 mmHg) by using the exhaust valve 113, and then the cuff pressure is decreased stepwise by a predetermined pressure decrement (for example, 5 mmHg) at regular time intervals. At this time, information on the cuff pulse wave detected by the cuff pressure detection unit 115 is obtained as the pulse wave information by the venous pressure measurement device 160.

After obtaining the pressure information and the pulse wave information, the venous pressure measurement device 160 determines whether the venous pressure of the subject can be estimated (step S102). Specifically, the determination is performed based on presence or absence of the noise component (for example, the noise components N1 and N2 of the lower graph of FIG. 6) and the candidate component (for example, the candidate component C of the lower graph of FIG. 6) in the pulse wave information.

When the pulse wave information does not include the noise component satisfying the predetermined condition and includes the candidate component, the venous pressure measurement device 160 determines that the venous pressure of the subject can be estimated (step S102: YES). At this time, the venous pressure measurement device 160 estimates the venous pressure of the subject (step S103), outputs information on the estimation result (step S104), and ends the process. Whether the pulse wave information includes the noise component satisfying the predetermined condition in is determined, for example, by determining whether an influence of the noise component is large in the pulse wave information by determining whether the magnitude of the noise component exceeds a threshold value, whether a period of the noise component exceeds a predetermined number of seconds, whether the number of times of detection of the noise component is large within a predetermined time, or the like.

On the other hand, when the pulse wave information includes the noise component satisfying the predetermined condition or the pulse wave information does not include the candidate component, the venous pressure measurement device 160 determines that the venous pressure of the subject cannot be estimated (step S102: NO). At this time, the venous pressure measurement device 160 determines whether the value range of the venous pressure of the subject can be calculated (step S105). The venous pressure measurement device 160 performs the determination based on, for example, the position, the period length, the number, the magnitude, and the like of the noise component.

For example, the venous pressure measurement device 160 determines that the value range of the venous pressure of the subject can be calculated (step S105: YES) when the pulse wave information does not include the noise component satisfying the predetermined condition and the candidate component (for example, the upper and lower graphs of FIG. 5). For example, the venous pressure measurement device 160 may also determine that the value range of the venous pressure of the subject can be calculated when the pulse wave information includes the noise component satisfying the predetermined condition and does not include the candidate component (for example, the upper and lower graphs of FIG. 8). In addition, the venous pressure measurement device 160 may also determine that the value range of the venous pressure of the subject can be calculated when the pulse wave information includes the noise component satisfying the predetermined condition and the candidate component (for example, the upper and lower graphs of FIG. 5). Whether the pulse wave information includes the noise component satisfying the predetermined condition is determined by determining whether the influence of the noise component is large in the pulse wave information, similarly to step S102.

When it is determined in step S105 that the value range of the venous pressure can be calculated, the venous pressure measurement device 160 calculates the value range of the venous pressure (step S106), outputs the information on the calculation result (step S107), and ends the process.

On the other hand, when it is determined in step S105 that the value range of the venous pressure cannot be calculated (step S105: NO), the venous pressure measurement device 160 outputs the information on the error result (step S108), and ends the process. For example, the venous pressure measurement device 160 may determine that the value range of the venous pressure of the subject cannot be calculated when the pulse wave information includes the noise component satisfying the predetermined condition and does not include the candidate component (for example, the upper and lower graphs of FIG. 9). Whether the pulse wave information includes the noise component satisfying the predetermined condition is determined by determining whether the influence of the noise component is large in the pulse wave information, similarly to step S102. In addition, the venous pressure measurement device 160 may determine that the value range of the venous pressure of the subject cannot be calculated when the waveform is not stable (for example, the upper and lower graphs of FIG. 10) even if the pulse wave information includes the noise component satisfying the predetermined condition and the candidate component.

Steps of the venous pressure measurement method illustrated in FIG. 11 may be performed in a different order, or a plurality of the steps may be performed at the same time. For example, step S102 and step S105 may be performed at the same time. In addition, it is also possible to firstly determine whether the error result should be output in consideration of a magnitude of noise or the like.

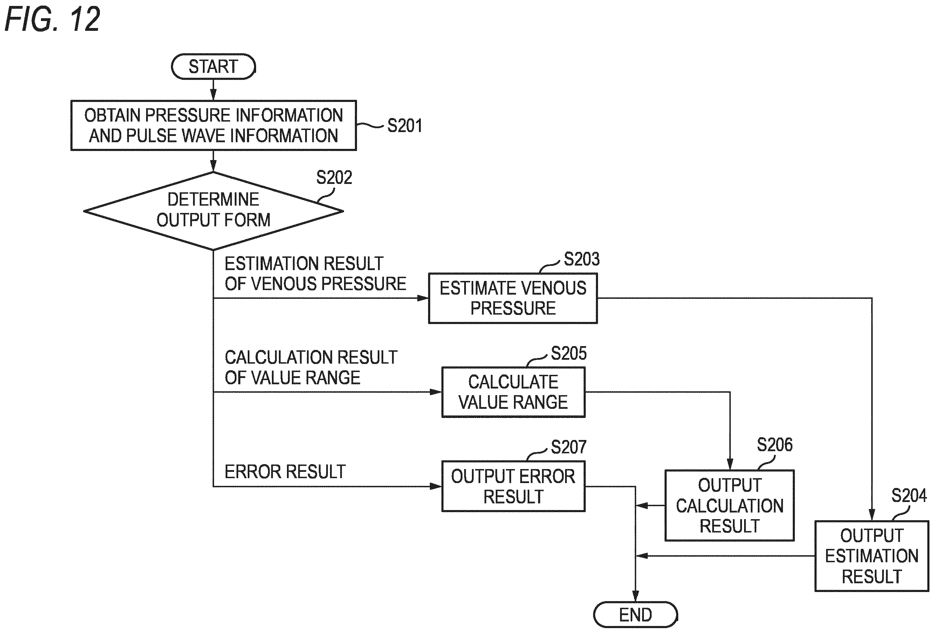

FIG. 12 is a flowchart illustrating another example of the venous pressure measurement method performed by the venous pressure measurement device 160. At this time, the venous pressure measurement device 160 can include, for example, a third determination unit that determines a form to be output, instead of the first determination unit 512 and the second determination unit 514.

The venous pressure measurement device 160 firstly obtains the pulse wave information and the pressure information in the same manner as described in step S101 of FIG. 11 (step S201), and then determines the form to be output (step S202). Specifically, in step S202, the venous pressure measurement device 160 analyzes the noise component and the candidate component of the pulse wave information obtained in step S201 so as to determine which one of the estimation result, namely a unique value, the calculation result of the value range of the venous pressure, and the error result is to be output. The venous pressure measurement device 160 determines the form to be output, for example, in the same manner as described in steps S102 and S105 of FIG. 11.

When it is determined in step S202 that the estimation result is to be output (step S202: estimation result), the venous pressure measurement device 160 estimates the unique value of the venous pressure based on the pulse wave information and the pressure information obtained in step S201 (step S203). Thereafter, the venous pressure measurement device 160 outputs the information on the estimation result (step S204), and ends the process.

On the other hand, when it is determined in step S202 that the calculation result is to be output (step S202: calculation result), the venous pressure measurement device 160 calculates the value range of the venous pressure based on the pulse wave information and the pressure information obtained in step S201 (step S205). Thereafter, the venous pressure measurement device 160 outputs the information on the estimation result (step S206), and ends the process.

In addition, when it is determined in step S202 that the error result is to be output (step S202: error result), the venous pressure measurement device 160 outputs the information on the error result (step S207), and ends the process.

### (Operational Effects of Venous Pressure Measurement Device and Venous Pressure Examination System)

According to the venous pressure measurement device 160 and the venous pressure examination system 100 of the presently disclosed subject matter, for example, when the venous pressure of the subject cannot be estimated due to an influence of noise or the like, the value range of the venous pressure of the subject is calculated based on the pulse wave information. As a result, for example, clinically useful information is increased, which can be used for medical treatment by a doctor. Hereinafter, this operational effect will be described.

Information on venous pressure such as central venous pressure can be an indicator of body congestion, and is non-invasively and quantitatively measured by using a cuff or the like. However, when venous pressure of a patient with congestion is measured through using a cuff or the like, there is a possibility that a signal derived from a venous pulse wave cannot be accurately obtained. This is because a respiratory condition of the patient with congestion tends to become unstable, and the signal derived from the venous pulse wave is affected by such respiration. In addition, there is a high possibility that a patient with heart failure also has arrhythmia. According to the method using the cuff or the like, there is a possibility that the signal derived from the venous pulse wave cannot be accurately obtained when arrhythmia occurs in the patient with heart failure. Further, it is difficult for a patient with difficulty breathing or the like to maintain a body thereof in a resting state for a certain period of time, and thus noise is likely to occur due to body movement.

According to the venous pressure measurement device 160 and the venous pressure measurement system 10, even when noise or the like is generated due to respiration, arrhythmia, or the like of the subject, the value range of the venous pressure is calculated. For example, when the venous pressure measurement device 160 determines the venous pressure of the subject to be equal to or higher than 10 mmHg or equal to or lower than 10 mmHg based on the pulse wave information, and information on the value range of the venous pressure is output, the doctor or the like can effectively use the information for diagnosing congestion of the subject.

In addition, according to the venous pressure measurement device 160 and the venous pressure measurement system 10, since it is determined whether the value range of the venous pressure can be calculated, reliability of the value range of the venous pressure can be maintained.

As described above, according to the venous pressure measurement device 160 and the venous pressure measurement system 10 of the present embodiment, when the venous pressure of the subject cannot be estimated, the value range of the venous pressure of the subject is calculated based on the pulse wave information. Therefore, the value range of the venous pressure can be calculated.

In addition, according to the venous pressure measurement system 10, since the pulse wave information on the pulse wave of the subject is obtained through using the cuff 111, it is possible to non-invasively measure the venous pressure of the subject.

As described above, in the embodiment, the venous pressure measurement device, the venous pressure measurement method, and the venous pressure measurement program according to the presently disclosed subject matter have been described. However, it is needless to say that addition, modification, and omission can be made as appropriate by those skilled in the art within the scope of the technical idea of the presently disclosed subject matter.

For example, although the pulse wave is measured by decreasing the cuff pressure in the above embodiment, the pulse wave may also be measured by increasing the cuff pressure.

In addition, although an example in which the pulse wave information is obtained through using the cuff 111 has been described in the above embodiment, the pulse wave information may also be obtained through using another sensor. For example, the pulse wave information may be obtained through using a photoelectric volume pulse wave sensor or the like.

Alternatively, the venous pressure may be calculated based on pulse wave information that is non-invasively measured by another system and obtained via a network interface from a server (computer) arranged on a communication network. Alternatively, a sensor including a light source and a photodetector may be disposed on a neck of the subject, and the venous pressure may be estimated by performing an operation using near-infrared spectroscopy (NIRS) on an optical signal obtained by the sensor.

In addition, in order to improve accuracy of pulse wave measurement, a plurality of sensors may be used to measure the pulse wave. For example, two of the cuffs 111 may be used, or the cuffs 111 and a photoplethysmogram sensor 70 may be used in combination. In addition, an electrode and an electrocardiogram measurement device may be further included, and a pulse wave of an electrocardiogram may be taken into consideration.

In addition, although an example in which whether the venous pressure of the subject can be estimated is determined based on the presence or absence of the noise component and the candidate component has been mainly described in the above embodiment, whether the venous pressure of the subject can be estimated may also be determined by using another method. For example, whether the venous pressure of the subject can be estimated may be determined by using a machine learning model.

In addition, although the functions of the controller 150 are divided into the obtaining unit 511, the first determination unit 512, the estimation unit 513, the second determination unit 514, the calculation unit 515, and the output unit 516 in the above-described embodiment, a part or all of the functions may be integrated. For example, the first determination unit 512, the estimation unit 513, the second determination unit 514, and the calculation unit 515 may be integrated, or the first determination unit 512 and the second determination unit 514 may be integrated.

In addition, ways and methods for performing various processes of the venous pressure measurement device 160 according to the above-described embodiment can be implemented by a dedicated hardware circuit or a programmed computer. The program may be provided by, for example, a computer-readable recording medium such as a compact disc read only memory (CD-ROM), or may be provided online via a network such as the Internet. In this case, the program recorded in the computer-readable recording medium is normally transferred to and stored in a storage unit such as a hard disk. In addition, the program may be provided as independent application software, or may be incorporated in software of the venous pressure measurement device 160 as a function thereof.

In addition, processing units of the flowchart in the above embodiment are divided according to main processing contents in order to facilitate understanding of each process. The presently disclosed subject matter is not limited by the way of classifying processing steps. Each process may be divided into a greater number of processing steps. In addition, one processing step may execute more processes.

According to the venous pressure measurement device, the venous pressure measurement method, and the venous pressure measurement program according to the presently disclosed subject matter, when the venous pressure of the subject cannot be estimated, the value range of the venous pressure of the subject is calculated based on the pulse wave information. Therefore, the value range of the venous pressure can be calculated.

## Claims

1. A venous pressure measurement device (160) comprising a controller (150) configured to:
obtain (S101) pulse wave information on a pulse wave of a subject measured by a sensor (114, 115);
determine (S102) whether the venous pressure of the subject can be estimated from the obtained pulse wave information,
determine (S105), when the venous pressure of the subject cannot be estimated, whether a value range of the venous pressure of the subject can be calculated based on the obtained pulse wave information; and
calculate (S106) a value range of venous pressure of the subject based on the obtained pulse wave information when it is determined that the venous pressure of the subject cannot be estimated and the value range of the venous pressure of the subject can be calculated from the obtained pulse wave information.

2. The venous pressure measurement device according to claim 1, wherein the controller is further configured to determine whether the venous pressure of the subject is estimatable from the pulse wave information based on a noise component affected by disturbance and a candidate component that is estimatable as a venous wave of the subject.

3. The venous pressure measurement device according to claim 2, wherein the controller is configured to determine that the venous pressure of the subject is not estimatable from the pulse wave information when the pulse wave information includes the noise component satisfying a predetermined condition or when the pulse wave information does not include the candidate component.

4. The venous pressure measurement device according to claim 2, wherein the controller is configured to estimate the venous pressure of the subject from the pulse wave information when the pulse wave information includes the candidate component and does not include the noise component satisfying a predetermined condition.

5. The venous pressure measurement device according to any one of claims 2 to 4, wherein the controller is further configured to determine whether the value range of the venous pressure of the subject is calculable based on the pulse wave information obtained by the obtaining unit.

6. The venous pressure measurement device according to claim 5, wherein the controller is configured to calculate the value range of the venous pressure of the subject when the pulse wave information does not include the noise component satisfying the predetermined condition and the candidate component.

7. The venous pressure measurement device according to claim 5, wherein the controller is configured to calculate the value range of the venous pressure of the subject or determine that an error has occurred when the pulse wave information includes the noise component satisfying the predetermined condition and does not include the candidate component.

8. The venous pressure measurement device according to claim 5, wherein the controller is configured to calculate the value range of the venous pressure of the subject or determine that an error has occurred when the pulse wave information includes the noise component satisfying the predetermined condition and the candidate component.

9. The venous pressure measurement device according to any one of claims 5 to 8, wherein the controller is configured to determine whether the value range of the venous pressure of the subject is calculable based on at least one of a position, a period length, a number, and a magnitude of the noise component.

10. The venous pressure measurement device according to any one of claims 2 to 9, wherein the sensor is a cuff attached to a predetermined portion of the subject, and
the controller is configured to obtain the pulse wave information based on pressure received by the cuff from the predetermined portion when cuff pressure of the cuff is changed.

11. The venous pressure measurement device according to claim 10, wherein the controller is configured to calculate the value range of the venous pressure of the subject based on the pulse wave information and pressure information on the cuff pressure.

12. The venous pressure measurement device according to claim 10 or 11, wherein the controller is configured to determine that the pulse wave information includes the candidate component when an amplitude of the pulse wave of the subject changes in a predetermined manner in accordance with a change in the cuff pressure.

13. The venous pressure measurement device according to any one of claims 1 to 12, wherein the controller is further configured to:
output any one of a calculation result regarding the value range of the venous pressure of the subject calculated by the controller, an estimation result regarding the venous pressure of the subject estimated from the pulse wave information, and an error result indicating that the estimation of the venous pressure of the subject and the calculation of the value range of the venous pressure of the subject are not performable.

14. A computer-implemented venous pressure measurement method comprising:
obtaining (S101) pulse wave information on a pulse wave of a subject measured by a sensor (114, 115);
determining (S102) whether the venous pressure of the subject can be estimated from the obtained pulse wave information;
determining (S105), when the venous pressure of the subject cannot be estimated, whether a value range of the venous pressure of the subject can be calculated based on the obtained pulse wave information; and
calculating (S106) a value range of venous pressure of the subject based on the obtained pulse wave information when it is determined that the venous pressure of the subject cannot be estimated and the value range of the venous pressure of the subject can be calculated from the obtained pulse wave information.

15. A program causing a computer to perform the venous pressure measurement method according to claim 14.

## Patentansprüche

1. Venendruck-Messvorrichtung (160), die eine Steuerungseinrichtung (150) umfasst, die ausgeführt ist zum:
Ermitteln (S101) von Pulswellen-Informationen über eine von einem Sensor (114, 115) gemessene Pulswelle eines Patienten;
Feststellen (S102), ob der Venendruck des Patienten anhand der ermittelten Pulswellen-Informationen geschätzt werden kann,
Feststellen (S105), ob ein Wertebereich des Venendrucks des Patienten auf Basis der ermittelten Pulswellen-Informationen berechnet werden kann, wenn der Venendruck des Patienten nicht geschätzt werden kann; und
Berechnen (S106) eines Wertebereiches von Venendruck des Patienten auf Basis der ermittelten Pulswellen-Informationen, wenn festgestellt wird, dass der Venendruck des Patienten nicht geschätzt werden kann, und der Wertebereich des Venendrucks des Patienten anhand der ermittelten Pulswellen-Informationen berechnet werden kann.

2. Venendruck-Messvorrichtung nach Anspruch 1, wobei die Steuerungseinrichtung des Weiteren so ausgeführt ist, dass sie auf Basis einer Rausch-Komponente, die durch Störung beeinflusst wird, sowie einer Kandidaten-Komponente, die als eine Venenwelle des Patienten geschätzt werden kann, feststellt, ob der Venendruck des Patienten anhand der Pulswellen-Informationen geschätzt werden kann.

3. Venendruck-Messvorrichtung nach Anspruch 2, wobei die Steuerungseinrichtung so ausgeführt ist, dass sie feststellt, dass der Venendruck des Patienten anhand der Pulswellen-Informationen nicht geschätzt werden kann, wenn die Pulswellen-Informationen die Rausch-Komponente einschließen, die eine vorgegebene Bedingung erfüllt, oder wenn die Pulswellen-Informationen die Kandidaten-Komponente nicht einschließen.

4. Venendruck-Messvorrichtung nach Anspruch 2, wobei die Steuerungseinrichtung so ausgeführt ist, dass sie den Venendruck des Patienten anhand der Pulswellen-Informationen schätzt, wenn die Pulswellen-Informationen die Kandidaten-Komponente einschließen und die Rausch-Komponente, die eine vorgegebene Bedingung erfüllt, nicht einschließen.

5. Venendruck-Messvorrichtung nach einem der Ansprüche 2 bis 4, wobei die Steuerungseinrichtung des Weiteren so ausgeführt ist, dass sie feststellt, ob der Wertebereich des Venendrucks des Patienten auf Basis der durch die Ermittlungs-Einheit ermittelten Pulswellen-Informationen berechnet werden kann.

6. Venendruck-Messvorrichtung nach Anspruch 5, wobei die Steuerungseinrichtung so ausgeführt ist, dass sie den Wertebereich des Venendrucks des Patienten berechnet, wenn die Pulswellen-Informationen die Rausch-Komponente, die die vorgegebene Bedingung erfüllt, und die Kandidaten-Komponente nicht einschließen.

7. Venendruck-Messvorrichtung nach Anspruch 5, wobei die Steuerungseinrichtung so ausgeführt ist, dass sie den Wertebereich des Venendrucks des Patienten berechnet oder feststellt, dass ein Fehler aufgetreten ist, wenn die Pulswellen-Informationen die Rausch-Komponente einschließen, die die vorgegebene Bedingung erfüllt, und die Kandidaten-Komponente nicht einschließen.

8. Venendruck-Messvorrichtung nach Anspruch 5, wobei die Steuerungseinrichtung so ausgeführt ist, dass sie den Wertebereich des Venendrucks des Patienten berechnet oder feststellt, dass ein Fehler aufgetreten ist, wenn die Pulswellen-Informationen die Rausch-Komponente, die die vorgegebene Bedingung erfüllt, sowie die Kandidaten-Komponente einschließen.

9. Venendruck-Messvorrichtung nach einem der Ansprüche 5 bis 8, wobei die Steuerungseinrichtung so ausgeführt ist, dass sie feststellt, ob der Wertebereich des Venendrucks des Patienten auf Basis einer Position, einer Periodenlänge, einer Anzahl oder/und eines Betrages der Rausch-Komponente berechnet werden kann.

10. Venendruck-Messvorrichtung nach einem der Ansprüche 2 bis 9, wobei der Sensor eine an einem vorgegebenen Abschnitt des Patienten angebrachte Manschette ist, und
die Steuerungseinrichtung so ausgeführt ist, dass sie die Pulswellen-Informationen auf Basis von durch die Manschette von dem vorgegebenen Abschnitt empfangenem Druck ermittelt, wenn sich Manschetten-Druck der Manschette ändert.

11. Venendruck-Messvorrichtung nach Anspruch 10, wobei die Steuerungseinrichtung so ausgeführt ist, dass sie den Wertebereich des Venendrucks des Patienten auf Basis der Pulswellen-Informationen sowie von Druck-Informationen über den Manschetten-Druck berechnet.

12. Venendruck-Messvorrichtung nach Anspruch 10 oder 11, wobei die Steuerungseinrichtung so ausgeführt ist, dass sie feststellt, dass die Pulswellen-Informationen die Kandidaten-Komponente einschließen, wenn eine Amplitude der Pulswelle des Patienten sich auf eine vorgegebene Art entsprechend einer Änderung des Manschetten-Drucks ändert.

13. Venendruck-Messvorrichtung nach einem der Ansprüche 1 bis 12, wobei die Steuerungseinrichtung des Weiteren ausgeführt ist zum:
Ausgeben eines durch die Steuerungseinrichtung berechneten Berechnungsergebnisses bezüglich des Wertebereiches des Venendrucks des Patienten, eines anhand der Pulswellen-Informationen geschätzten Schätzungsergebnisses bezüglich des Venendrucks des Patienten und/oder eines Fehlerergebnisses, das anzeigt, dass die Schätzung des Venendrucks des Patienten und die Berechnung des Wertebereiches des Venendrucks des Patienten nicht durchgeführt werden können.

14. Computerimplementiertes Venendruck-Messverfahren, das umfasst:
Ermitteln (S101) von Pulswellen-Informationen über eine von einem Sensor (114, 115) gemessene Pulswelle eines Patienten;
Feststellen (S102), ob der Venendruck des Patienten anhand der ermittelten Pulswellen-Informationen geschätzt werden kann;
Feststellen (S105), ob ein Wertebereich des Venendrucks des Patienten auf Basis der ermittelten Pulswellen-Informationen berechnet werden kann, wenn der Venendruck des Patienten nicht geschätzt werden kann; und
Berechnen (S106) eines Wertebereiches von Venendruck des Patienten auf Basis der ermittelten Pulswellen-Informationen, wenn festgestellt wird, dass der Venendruck des Patienten nicht geschätzt werden kann, und der Wertebereich des Venendrucks des Patienten anhand der ermittelten Pulswellen-Informationen berechnet werden kann.

15. Programm, das einen Computer veranlasst, das Venendruck-Messverfahren nach Anspruch 14 durchzuführen.

## Revendications

1. Dispositif de mesure de pression veineuse (160) comprenant un dispositif de commande (150) configuré pour :
obtenir (S101) des informations d'onde d'impulsions d'une onde d'impulsions d'un sujet mesurée par un capteur (114, 115) ;
déterminer (S102) si la pression veineuse du sujet peut être estimée à partir des informations d'onde d'impulsions obtenues,
déterminer (S105), lorsque la pression veineuse du sujet ne peut pas être estimée, si une plage de valeurs de la pression veineuse du sujet peut être calculée sur la base des informations d'onde d'impulsions obtenues ; et
calculer (S106) une plage de valeurs de la pression veineuse du sujet sur la base des informations d'onde d'impulsions obtenues lorsqu'il est déterminé que la pression veineuse du sujet ne peut pas être estimée et que la plage de valeurs de la pression veineuse du sujet peut être calculée à partir des informations d'onde d'impulsions obtenues.

2. Dispositif de mesure de pression veineuse selon la revendication 1, dans lequel le dispositif de commande est en outre configuré pour déterminer si la pression veineuse du sujet peut être estimée à partir des informations d'onde d'impulsions sur la base d'une composante de bruit affectée par une perturbation et d'une composante candidate qui peut être estimée comme onde veineuse du sujet.

3. Dispositif de mesure de pression veineuse selon la revendication 2, dans lequel le dispositif de commande est configuré pour déterminer que la pression veineuse du sujet ne peut pas être estimée à partir des informations d'onde d'impulsions lorsque les informations d'onde d'impulsions incluent la composante de bruit satisfaisant une condition prédéterminée ou lorsque les informations d'onde d'impulsions n'incluent pas de composante candidate.

4. Dispositif de mesure de pression veineuse selon la revendication 2, dans lequel le dispositif de commande est configuré pour estimer la pression veineuse du sujet à partir des informations d'onde d'impulsions lorsque les informations d'onde d'impulsions incluent la composante candidate et n'incluent pas la composante de bruit satisfaisant une condition prédéterminée.

5. Dispositif de mesure de pression veineuse selon l'une quelconque des revendications 2 à 4, dans lequel le dispositif de commande est en outre configuré pour déterminer si la plage de valeurs de la pression veineuse du sujet peut être calculée sur la base des informations d'onde d'impulsions obtenues par l'unité d'obtention.

6. Dispositif de mesure de pression veineuse selon la revendication 5, dans lequel le dispositif de commande est configuré pour calculer la plage de valeurs de la pression veineuse du sujet lorsque les informations d'onde d'impulsions n'incluent pas la composante de bruit satisfaisant la condition prédéterminée et la composante candidate.

7. Dispositif de mesure de pression veineuse selon la revendication 5, dans lequel le dispositif de commande est configuré pour calculer la plage de valeurs de la pression veineuse du sujet ou déterminer qu'une erreur s'est produite lorsque les informations d'onde d'impulsions incluent la composante de bruit satisfaisant la condition prédéterminée et n'incluent pas la composante candidate.

8. Dispositif de mesure de pression veineuse selon la revendication 5, dans lequel le dispositif de commande est configuré pour calculer la plage de valeurs de la pression veineuse du sujet ou déterminer qu'une erreur s'est produite lorsque les informations d'onde d'impulsions incluent la composante de bruit satisfaisant la condition prédéterminée et la composante candidate.

9. Dispositif de mesure de pression veineuse selon l'une quelconque des revendications 5 à 8, dans lequel le dispositif de commande est configuré pour déterminer si la plage de valeurs de la pression veineuse du sujet peut être calculée sur la base d'au moins l'un·e parmi une position, une longueur de période, un nombre, et une magnitude de la composante de bruit.

10. Dispositif de mesure de pression veineuse selon l'une quelconque des revendications 2 à 9, dans lequel le capteur est un coussinet fixé à une portion prédéterminée du sujet, et
le dispositif de commande est configuré pour obtenir les informations d'onde d'impulsions sur la base de la pression reçue par le coussinet depuis la portion prédéterminée lorsque la pression de coussinet du coussinet est changée.

11. Dispositif de mesure de pression veineuse selon la revendication 10, dans lequel le dispositif de commande est configuré pour calculer la plage de valeurs de la pression veineuse du sujet sur la base des informations d'onde d'impulsions et des informations de pression sur la pression du coussinet.

12. Dispositif de mesure de pression veineuse selon la revendication 10 ou 11, dans lequel le dispositif de commande est configuré pour déterminer que les informations d'onde d'impulsions incluent la composante candidate lorsqu'une amplitude de l'onde d'impulsions du sujet change d'une manière prédéterminée selon un changement au niveau de la pression du coussinet.

13. Dispositif de mesure de pression veineuse selon l'une quelconque des revendications 1 à 12, dans lequel le dispositif de commande est en outre configuré pour :
délivrer n'importe lequel d'un résultat de calcul concernant la plage de valeurs de la pression veineuse du sujet calculée par le dispositif de commande, d'un résultat d'estimation concernant la pression veineuse du sujet estimé à partir des informations d'onde d'impulsions, et d'un résultat d'erreur indiquant que l'estimation de la pression veineuse du sujet et le calcul de la plage de valeurs de la pression veineuse du sujet ne peuvent pas être exécutés.

14. Procédé de mesure de pression veineuse mis en œuvre par ordinateur comprenant les étapes consistant à :
obtenir (S101) des informations d'onde d'impulsions sur une onde d'impulsions d'un sujet mesurée par un capteur (114, 115) ;
déterminer (S102) si la pression veineuse du sujet peut être estimée à partir des informations d'onde d'impulsions obtenues ;
déterminer (S105), lorsque la pression veineuse du sujet ne peut pas être estimée, si une plage de valeurs de la pression veineuse du sujet peut être calculée sur la base des informations d'onde d'impulsions obtenues ; et
calculer (S106) une plage de valeurs de la pression veineuse du sujet sur la base des informations d'onde d'impulsions obtenues lorsqu'il est déterminé que la pression veineuse du sujet ne peut pas être estimée et que la plage de valeurs de la pression veineuse du sujet peut être calculée à partir des informations d'onde d'impulsions obtenues.

15. Programme amenant un ordinateur à effectuer le procédé de mesure de pression veineuse selon la revendication 14.
